# EUROPEAN PATENT APPLICATION

(11) **EP 0 826 803 A2**
(43) Date of publication of application: **04.03.1998**
(21) Application number: 97402004.2
(22) Date of filing: 27.08.1997
(51) Int. Cl.: D01F 6/92, D01F 6/84, C08L 67/04, C08G 63/08, A61L 17/00

(54) **Biodegradable polyester fiber**

(30) Priority: 27.08.1996 JP 225463/96
(71) Applicant: Takasago International Corporation, Tokyo 108 (JP)
(72) Inventor: Gonda, Yoshiharu, Hiratsuka-shi, Kanagawa-Ken (JP); Hori, Yoji, Hiratsuka-shi, Kanagawa-Ken (JP); Hongo, Hideyuki, Hiratsuka-shi, Kanagawa-Ken (JP); Hagiwara, Toshimitsu, Hiratsuka-shi, Kanagawa-Ken (JP); Aoki, Yoko (née Takahashi), Hiratsuka-shi, Kanagawa-Ken (JP)
(74) Representative: Uchida, Kenji

(57) **Abstract**

The present invention provides a fiber comprising a biodegradable polymer. The present invention further provides a fiber comprising a biodegradable polymer composition containing a 3-hyroxybutyric acid moiety in the molecule thereof, which retains the properties of poly(3-hyroxybutyric acid) and has a high strength and a high melting point so as to be tolerant in practical use and also provides a process for preparing the fiber by melt spinning. Specifically, the fiber comprises a biodegradable polymer composition comprising a copolymerized polyester containing a stereospecific poly(3-hydoxybutyric acid) unit, a random copolymerized polyester containing poly(3-hydoxybutyric acid) or 3-hydoxybutyric acid units, and optionally a polyester containing specific units, and a process for preparing the fiber by melt spinning.

## Description

The present invention relates to a fiber comprising a biodegradable polymer and a fiber comprising a composition containing the same polymer. The present invention further relates to a process for preparing the fiber. More specifically, the present invention relates to a fiber comprising a biodegradable polymer composition which can be used for fishing lines, fishing nets, agricultural nets, and the like as well as for living necessaries such as women's sanitary goods, masks, wet tissue paper, under wears, towels, handkerchiefs, kitchen towels, diapers, and the like, and also relates to a process for preparing the fiber.

### Description of the Related Art

As a polymer composition used for fishing lines, fishing nets, agricultural nets, living necessaries, and the like there has been conventionally used a composition comprising polyamide, polyester, vinylon, polyolefin, and the like. However, articles made of these polymer compositions have a disadvantage in that, because of the poor degradability thereof, they tend to pollute the environment when used articles are discarded in the natural environment. In order to solve this disadvantage incineration, recovery, regeneration, and the like of the used articles are required, which entails enormous cost. In fact, since the recovery of the used articles is difficult, they are mostly left in the natural environment, causing environmental destruction.

As one of the means for solving such problems, there is a method of utilizing a polymer composition which can be easily degraded by microorganisms existing in nature.

For example, Japanese Patent Application Laid-Open No. 1-175855 discloses a surgical suture made of polycaprolactone and Japanese Patent Application Laid-Open No. 5-78912 discloses a monofilament made of polypropiolactone. However, the use of polycaprolactone and polypropiolactone are restricted because the melting points thereof are low, i.e., about 60°C and about 97°C, respectively.

On the other hand, as examples of polymer compositions having a higher melting point, Japanese Patent Application Laid-Open No. 45-31696 discloses a surgical suture made of polylactide and copolymer thereof, and Biomaterials, 1987, Vol. 8, 129 describes a biodegradable polyester fiber using a random copolymerized polyester containing microorganism-produced 3-hydroxybutyric acid units. However, the use of a polylactide-based compound is restricted because it is susceptible to moisture and tends to easily deteriorate, while the aforementioned polyester fibers have problems in that not only do they need improvement in strength, but also the cost is high since they are produced by microorganisms.

It has been reported that, in the melt spinning of microorganism-produced poly(3-hyroxybutyric acid), the polymer deforms like rubber at the step of extending the fiber after melting and extrusion when the polymer is not crystallized, while the polymer is brittle and breaks at any temperature regardless of the stress applied when the crystallinity is high. Thus, the obtained fiber is brittle and significantly poor in strength (Elsevier Applied Science, London, pp. 33-43, 1988).

Accordingly, an object of the present invention is to provide a fiber comprising a biodegradable polymer containing a 3-hydroxybutyric acid moiety in the molecule thereof, which retains the properties of poly(3-hydroxybutyric acid) and has a high strength and a high melting point so as to be tolerant in practical use.

Another object of the present invention is to provide a fiber comprising a biodegradable polymer composition containing the polymer.

Another object of the present invention is to provide a fiber comprising a high biodegradable polymer composition, which comprises a specific block copolymerized polyester containing stereospecific poly(3-hydroxybutyric acid) units; at least one polyester selected from the group consisting of poly(3-hydroybutyric acid) and a random copolymerized polyester containing 3-hydroxybutyric acid units; and optionally a specific polyester. This polymer composition has a high strength, and the melting point can be optionally controlled in the range from 60°C to 180°C, and extensibility can also be controlled.

The present invention provides a fiber comprising
(1) a biodegradable polymer characterized by containing:
   a block copolymerized polyester containing a stereospecific poly(3-hydroxybutyric acid) unit, in which a molar ratio of the structural unit (I) to the structural unit (II) represented by the following general formulae is 10-90:90-10, and a number-average molecular weight thereof is from 20,000 to 1,000,000: 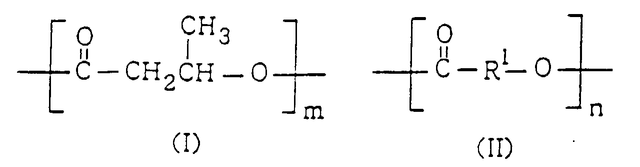 wherein, R¹ is a divalent hydrocarbon group having from 2 to 14 carbon atoms which may contain an oxygen atom or double bond; m and n are each an integer of from 100 to 9,000, and m+n is of from 200 to 10,000.
   The present invention provides a fiber comprising a biodegradable polymer composition: comprising a block copolymerized polyester described above: and poly(3-hydroybutyric acid) or a random copolymerized polyester containing 3-hydroxybutyric acid units.
(2) The present invention provides a fiber comprising a biodegradable polymer composition as described in (1) above, characterized by further containing a polyester having a structural unit (II) represented by the general formula above.
(3) The present invention also provides a process for preparing a fiber comprising a biodegradable polymer composition as described in (1) or (2) above, in which melt spinning is carried out at a temperature from 150 to 220°C and extension is carried out at a temperature of from 10 to 150°C.

According to the present invention, a preferred polymer composition includes a polymer composition containing a block copolymerized polyester having a stereospecific poly(3-hydroxybutyric acid) unit (I) and unit (II) of the above formula; and at least one polyester selected from the group consisting of poly(3-hydroybutyric acid) and a random copolymerized polyester containing 3-hydroxybutyric acid units. The above polymer composition may further comprise a polyester having a structure as defined for the structure unit (II). In the above compositions, the disadvantage that poly(3-hydroxybutyric acid) is low in extensibility and brittle is minimized. By melt spinning the biodegradable polymer composition and extending it under appropriate conditions, a polyester fiber can be produced with high strength and excellent biodegradability and hydrolyzing property in which the melting point thereof can be optionally changed and extensibility can also be controlled. Here, the melting point and extensibility are controlled as follows:

For example, a fiber which is elastic when stretched is formed by using a soft polymer such as polycaprolactone for the structural unit (II). Similarly, an extensible fiber is formed by using a rubber-like polymer for the structural unit (II). A fiber having low extensibility is formed by using a hard polymer such as polylactide for the structural unit (II).

The present invention will be explained in detail hereinafter.

First, an explanation is given of the block copolymerized polyester constituting the biodegradable polymer composition of the present invention.

The block copolymerized polyester is a block copolymerized polyester containing stereospecific isotactic or syndiotactic poly(3-hydroxybutyric acid) as structural units (I) in the total structure represented by the following general formulae: 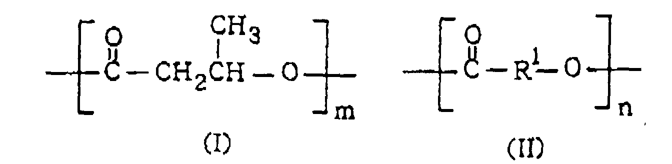 in which a molar ratio of the structural unit (I) to the structural unit (II) is 10-90:90-10, preferably 20-80:80-20, and which has a number-average molecular weight of from 20,000 to 1,000,000. In the general formulae, R¹ is a divalent hydrocarbon group having from 2 to 14 carbon atoms which may contain an oxygen atom or a double bond; m and n are each an integer of from 100 to 9,000, and m+n is from 200 to 10,000, preferably from 1,000 to 10,000.

As the structural unit (I) of the block copolymerized polyester, a structural unit derived from optically active (R)-β-butyrolactone (abbreviated as (R)-β-BL, hereinafter), (S)-β-butyrolactone (abbreviated as (S)-β-BL, hereinafter), or racemic β-butyrolactone (abbreviated as β-BL, hereinafter) is preferred. That is, an oligomer or polymer obtained by ring-opening polymerization of (R)-β-BL or (S)-β-BL is preferred.

(R)-β-BL or (S)-β-BL used herein is easily prepared, for example, by a method disclosed by the present applicant in Japanese Patent Application Laid-Open Nos. 6-128245, 7-188201 and 7-206885, that is, by asymmetrically hydrogenating a diketene in the presence of ruthenium-optical active phosphine complex as a catalyst. β-BL is commercially available.

Next, an explanation is given of the structural unit (II) of the block copolymerized polyester.

R¹ of the aforementioned structural unit (II) is a divalent hydrocarbon group having from 2 to 14 carbon atoms which may contain an oxygen atom or a double bond. The structural unit (II) is preferably an oligomer or polymer obtained by ring-opening polymerization of one or a pluarlity of lactones containing R¹.

Specific examples of lactones which may be used in the present invention are β-propiolacton, β-butyrolactone (β-BL), β-ethyl-β-propiolactone, α-methyl-β-propiolactone, γ-butyrolactone, α-methyl-γ-butyrolactone, β-methyl-γ-butyrolactone, γ-methyl-γ-butyrolactone, δ-valerolactone, β-methyl-δ-valerolactone, ε-caprolactone, 15-pentadecanolide, 16-hexadecanolide, and the like. Specific examples of lactones in which the divalent organic group is an alkenyl group having from 2 to 14 carbon atoms and a double bond are 5,6-dihydro-2H-pyran-2-one, 3,4-dihydro-6-methyl-2H-pyran-2-one, 5,6-dihydro-6-methyl-2H-pyran-2-one, 9-hexadecene-16-olide, and the like. Specific examples of lactones in which the divalent organic group is an alkyl group having from 2 to 14 carbon atoms and including an ester group or ether group are glycolide, L-lactide, DL-lactide, 1,4-dioxepane-5-one, 7-methyl-1,4-dioxepane-5-one, 12-oxa-16-hexadecanolide, 11-oxa-16-hexadecanolide, 10-oxa-16-hexadecanolide, and the like.

These lactones may be produced by a known process and are commercially available. When the lactones have an asymmetric carbon atom, an optically active or racemic lactone may be used alone or in a combination of two or more thereof.

The block copolymerized polyester used in the present invention can be prepared using a known method, and specifically, can be prepared by the following method: A reaction vessel is charged with (R)-β-BL, (S)-β-BL, or β-BL in the presence of an inactive solvent or in the absence of a solvent in an atmosphere of an inert gas such as nitrogen or argon. A polymerization catalyst described below is added thereto, and then, the reaction is carried out at a temperature of 60-180°C at atmospheric pressure for 30 minutes to 5 hours to complete the first step of polymerization. Then, after a small quantity of inactive solvent is added to the solution to decrease the viscosity, lactone other than (R)-β-BL, (S)-β-BL, or β-BL used in the first step is added, and the reaction is further carried out for 1 to 48 hours to complete the second step of polymerization. Accordingly, AB-type block copolymerized polyester can be obtained.

A BA-type block copolymerized polyester can be obtained by polymerizing lactone except for (R)-β-BL, (S)-β-BL or β-BL in the firs t step, adding (R)-β-BL, (S)-β-BL or β-BL in the reaction mixture and polymerizing the (R)-β-BL, (S)-β-BL or β-BL in the second step.

Further an ABA-type block copolymerized polyester can be obtained by adding (R)-β-BL, (S)-β-BL or β-BL in the reaction mixture containing the AB-type block copolymerized polyester and polymerizing the (R)-β-BL, (S)-β-BL or β-BL.

A BAB-type block copolymerized polyester can be obtained by the same process, i.e. the lactone except for (R)-β-BL, (S)-β-BL or β-BL is added in the reaction mixture containing the BA-type block copolymerized polyester and polymerizing the lactone.

Further an ABC-type block copolymerized polyester can be obtained by adding lactone except for the lactone which is used to produce the AB-type block copolymerized polyester in the reaction mixture containing the AB-type block copolymerized polyester and polymerizing the lactone.

A BAC-type block copolymerized polyester can be obtained by the same process, i.e. lactone except for the lactone which is used to produce the BA-type block copolymerized polyester is added in the reaction mixture containing the BA-type block copolymer and polymerizing the lactone added.

Examples of the catalyst which may be used in the polymerization reaction are stannum-based catalysts such as dibutyltin (IV) oxide, dioctyltin (IV) oxide, tin (II) dioctylate, dibutyltin (VI) dilaurate, and the like. In particular, a distannoxane catalyst represented by the following general formula (III) is preferred: 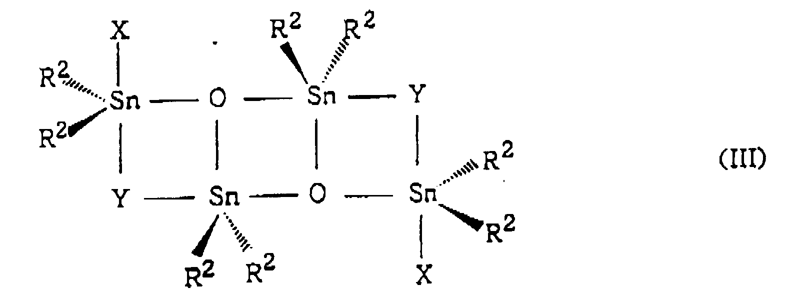 wherein, R² is an alkyl group having 1 to 12 carbon atoms, or an aralkyl or phenyl group ; X is selected from the group consisting of Cl, Br, and NCS; Y is selected from the group consisting of Cl, Br, NCS, OH, and an alkoxy group having from 1 to 4 carbon atoms and phenoxy group.

Examples of the distannoxane catalyst include 1-hydroxy-3-chlorotetrabutyl distannoxane, 1-hydroxy-3-chlorotetraoctyl distannoxane, 1-ethoxy-3-chlorotetrabutyl distannoxane, 1-ethoxy-3-chlorotetraoctyl distannoxane, 1-hydroxy-3-(isothiocyanate)tetrabutyl distannoxane, 1-hydroxy-3-(isothiocyanate)tetraoctyl distannoxane, 1-ethoxy-3-(isothiocyanate)tetrabutyl distannoxane, and 1-ethoxy-3-(isothiocyanate)tetraoctyl distannoxane.

These catalysts, for example, 1,3-dichlorotetraphenyl distannoxane and 1-hydroxy-3-(isothiocyanate)tetrabutyl distannoxane, can be easily synthesized by reacting dibutyltin (IV) oxide with dibutyltin (IV) diisothiocyanate in ethanol as described in J. Organomet. Chem. 3, p. 70 (1965) and J. Org. Chem. 56, p. 5307, (1991), respectively. These catalysts may be used alone or in a combination thereof,

Next, an explanation is given on the poly(3-hydroybutyric acid) or a random copolymerized polyester containing 3-hydroxybutyric acid units, which constitutes a component of the biodegradable polymer composition of the present invention. As used herein, the term "containing 3-hydroxybutyric acid units" means containing 3-hydroxybutyric acid units of 70 % or more. The remaining 30 % or less may be any copolymerizable compopnent, as long as the polyester retains biodegradability.

Specific examples of the random copolymerized polyester containing poly(3-hydroxybutyric acid) or 3-hydroxybutyric acid units which constitutes the biodegradable polymer composition of the present invention are microorganism-produced poly(3-hydroxyalkanoate) such as random copolymerized polyesters of poly(3-hydroxybutyric acid) and poly(3-hydroxypropanoic acid) [hereinafter abbreviated as poly(3-hydroxybutyric acid-co-3-hydroxypropanoic acid), the same shall apply hereinafter], poly(3-hydroxybutyric acid-co-3-hydroxypentanoic acid), poly(3-hydroxybutyric acid-co-4-hydroxybutyric acid), poly(3-hydroxybutyric acid-co-3-hydroxyhexanoic acid), poly(3-hydroxybutyric acid-co-3-hydroxyheptanoic acid), poly(3-hydroxybutyric acid-co-3-hydroxyoctanoic acid), and the like; and chemically synthesized poly(3-hydroxyalkanoate) such as poly(3-hydroxybutyric acid-co-3-hydroxypropanoic acid), poly(3-hydroxybutyric acid-co-3-hydroxypentanoic acid), poly(3-hydroxybutyric acid-co-4-hydroxybutyric acid), poly(3-hydroxybutyric acid-co-5-hydroxypentanoic acid), poly(3-hydroxybutyric acid-co-3-methyl-5-hydroxypentanoic acid), poly(3-hydroxybutyric acid-co-6-hydroxyhexanoic acid), poly(3-hydroxybutyric acid-co-15-hydroxypentadecanoic acid), poly(3-hydroxybutyric acid-co-L-lactide), poly(3-hydroxybutyric acid-co-7-methyl-1,4-dioxepane-5-one), poly(3-hydroxybutyric acid-co-12-oxa-16-hexadecanolide), and the like.

The random copolymerized polyester containing poly(3-hydroxybutyric acid) or 3-hydroxybutyric acid units which is chemically synthesized may be preferably prepared by using the aforementioned catalysts.

That is, the aforementioned random copolymerized polyester may be prepared by using the methods disclosed by the present applicant in Japanese Patent Application Laid-Open Nos. 6-256482 and 6-329768. Specifically, (R)-β-BL, (S)-β-BL, or β-BL and other lactones are simultaneously charged into a reaction vessel in the presence of inactive solvent or in the absence of solvent in an atmosphere of inert gas such as nitrogen or argon, then the aforementioned catalyst is added for reaction at a temperature of 60-100°C at atmospheric pressure for one hour to 3 days to obtain a copolymerized polyester.

Other specific examples of lactones used for preparing the copolymerized polyester in the present invention are β-propiolactone, β-butyrolactone (β-BL), β-ethyl-β-propiolactone, α-methyl-β-propiolactone, γ-butyrolactone, α-methyl-γ-butyrolactone, β-methyl-γ-butyrolactone, γ-methyl-γ-butyrolactone, δ-valerolactone, β-methyl-δ-valerolactone, ε-caprolactone, 15-pentadecanolide, 16-hexadecanolide, and the like. Examples of lactones in which the divalent organic group is an alkenyl group having from 2 to 14 carbon atoms and double bond include 5,6-dihydro-2H-pyran-2-one, 3,4-dihydro-6-methyl-2H-pyran-2-one, 5,6-dihydro-6-methyl-2H-pyran-2-one, 9-hexadecene-16-olide, and the like. Further, examples of lactones in which the divalent organic group is an alkyl group having from 2 to 14 carbon atoms and including an ether group or ester group are glycolide, L-lactide, DL-lactide, 1,4-dioxepane-5-one, 7-methyl-1, 4-dioxepane-5-one, 1,2-oxa-16-hexadecanolide, 11-oxa-16-hexadecanolide, 10-oxa-16-hexadecanolide, and the like.

These lactones may be produced by a known process and are commercially available. When lactones have an asymmetric carbon atom, an optical active or racemic lactone may be used alone or in a combination of two or more thereof.

An explanation is given of the polyester having the structural unit (II) which constitutes the biodegradable polymer composition of the present invention, hereinafter.

The polyester having the structural unit (II) which constitutes an optional component of the biodegradable polymer composition of the present invention is commercially available, or may be easily produced by using the aforementioned stannum-catalysts or catalyst conventionally used for ring-opening polymerization of lactones or polycondensation of hydroxycarboxylic acid and esters thereof such as diethylzinc-water, triethyl aluminum-water, ethylalumoxane, aluminum isopropoxide, titanium isopropoxide, and the like.

Specific examples of lactones used in the present invention are β-propiolactone, β-butyrolactone (β-BL), β-ethyl-β-propiolactone, α-methyl-β-propiolactone, γ-butyrolactone, α-methyl-γ-butyrolactone, β-methyl-γ-butyrolactone, γ-methyl-γ-butyrolactone, δ-valerolactone, β-methyl-δ-valerolactone, ε-caprolactone, 15-pentadecanolide, 16-hexadecanolide, and the like. Examples of lactones in which the divalent organic group is an alkenyl group having from 2 to 14 carbon atoms and double bond are 5,6-dihydro-2H-pyran-2-one, 3,4-dihydro-6-methyl-2H-pyran-2-one, 5,6-dihydro-6-methyl-2H-pyran-2-one, 9-hexadecene-16-olide, and the like. Examples of lactones in which the divalent organic group is an alkyl group having from 2 to 14 carbon atoms and including an ether group or ester group are glycolide, L-lactide, DL-lactide, 1,4-dioxepane-5-one, 7-methyl-1,4-dioxepane-5-one, 12-oxa-16-hexadecanolide, 11-oxa-16-hexadecanolide, 10-oxa-16-hexadecanolide, and the like. These lactones may be produced by a known process and are commercially available.

Specific examples of hydroxycarboxylic acids and esters thereof include glucolic acid, lactic acid, 3-hydroxypropionic acid, 3-hydroxybutyric acid, 3-hydroxyvaleric acid, 2-methyl-3-hydroxypropionic acid, 4-hydroxybutyric acid, 2-methyl-4-hydroxybutyric acid, 3-methyl-4-hydroxybutyric acid, 4-methyl-4-hydroxybutyric acid, 5-hydroxypentanoic acid, 3-methyl-5-hydroxypentanoic acid, 6-hydroxyhexanoic acid, 15-hydroxypentadecanoic acid, 16-hydroxyhexadecanoic acid, and the like. Examples of hydroxycarboxylic acids in which the divalent organic group of R¹ in the structural formula (II) is an alkenyl group having from 2 to 14 carbon atoms and a double bond are 5-hydroxy-3-pentenoic acid, 5-hydroxy-5-methyl-3-pentenoic acid, 16-hydroxy-9-hexadecenoic acid, and the like. Further, examples of hydroxycarboxylic acids in which the divalent organic group of R¹ in the structural formula (II) is an alkyl group having from 2 to 14 carbon atoms and including an ether group include 4-oxa-6-hydroxyhexanoic acid, 3-methyl-4-oxa-6-hydroxyhexanoic acid, 16-hydroxy-12-oxadecanoic acid, 16-hydroxy-11-oxahexadecanoic acid, 16-hyroxy-10-oxahexadecanoic acid, and the like; and esters corresponding to the aforementioned carboxylic acids.

When the lactones or hydroxycarboxylic acids and esters thereof have an asymmetric carbon atom, an optically active or racemic one may be used alone or in a combination of two or more thereof.

In the biodegradable polymer composition according to the present invention, the weight ratio the block copolymerized polyester to the random copolymerized polyester containing poly(3-hydroxybutyric acid) or 3-hydroxybutyric acid units may be preferably 10 - 80:20 - 90, more preferably 20-60:40-80. When the biodegradable polymer composition contains the optional polyester having the structural unit (II), the weight ratio of the block copolymerized polyester, random copolymerized polyester containing poly(3-hydroxybutyric acid) or 3-hydroxybutyric acid units and the polyester having the structural unit (II) may be preferably 10-50:40-80:10-50. It is economically advantageous to include the aforementioned polyester having the structural unit (II) in the biodegradable polymer composition of the present invention since the amount of block copolymerized polyester is reduced. Thus, if the amount of the polyester having the structural unit (II) is too small, the amount of the block copolymerized polyester is required to be increased, which raises cost as well as decreases extensibility and strength. On the contrary, if the amount of the polyester having the structural unit (II) is too large, the biodegradability may deteriorate.

In the biodegradable polymer composition according to the present invention, if the random copolymerized polyester containing poly(3-hydroxybutyric acid) or 3-hydroxybutyric acid units is less than 20 % by weight, the biodegradability of fiber may significantly deteriorate. On the contrary, if it is more than 90% by weight, it becomes hard to extend the fiber and the strength of fiber may also significantly deteriorate.

To the biodegradable polymer composition according to the present invention may be added a compounding agent conventionally used such as a stabilizer, a colorant, and the like. In order to increase the recrystallization rate and improve the workability, a nucleating agent such as boron nitride, titanium oxide, micromica, chalk, and the like may be added, if necessary, in an amount of 0.01-1% by weight.

Next, an explanation is given of the process for preparing the fiber comprising the biodegradable polymer composition according to the present invention.

In the melt spinning of the biodegradable polymer composition of the present invention, a spinneret having an aperture or apertures of from 0.3 to 1.0 mm for monofilament or multifilaments may be used. A spinneret having larger aperture may be used if necessary. The temperature of the spinneret is suitably from 10 to 200°C, preferably from 50 to 160°C. The melting portion for the polymer composition in the spinning apparatus is kept from 150 to 220°C.
If the temperature of the melting portion is less than 150°C, melt extrusion becomes difficult. On the contrary, if it exceeds 220°C, the polymer may be significantly degraded, and it becomes difficult to obtain a fiber with high strength.

Then, the melt-spun fiber is continuously extended after winding or without winding. The extension process may be carried out at room temperature, or by means of hot air, a hot plate, or a hot pin, or in the presence of a hot solvent such as water, glycerin, ethylene glycol, silicone oil, and the like at a temperature of from 10 to 150 °C, and preferably from room temperature to 120°C. The range of the temperature is determined as follows: If a polycaprolactone having low Tg (-60°C) and low Tm (60°C) is employed for the structural unit (II), for example, the extension needs to be carried out at a relative low temperature, while if polylactic acid (polylactide) having high Tg (about 53°C) and high Tm (180°C) is employed for the structural unit (II), the extension needs to be carried out at a relatively high temperature. The extension is carried out at a temperature below the melting point of the biodegradable polymer composition of the present invention with an extension ratio of 5 to 10 times. If the extension ratio is less than 5 times, an increase in the strength is small, while if it exceeds 10 times, the fiber tends to be frequently cut.

The fiber which has been extended in the above-described manner is subjected to heat treatment at a temperature of from 10 to 150 °C, if necessary.

Although the size of the fiber of the present invention may vary depending on the use thereof, the size is generally 50 d or more. The fiber of the present invention may be formed as desired in various shapes such as monofilament, multifilaments, cover spun yarn, and the like.

### EXAMPLE

The present invention will be explained in more detail hereinafter with reference to examples and test examples. However, the present invention is not intended to be limited thereto.

Analytical instruments used in the examples and test examples and instruments used in a biodegradation test are listed below.
(1) Nuclear magnetic resonance spectrum (NMR): AM-400 type (400MHz), manufactured by Bluker Co, Ltd.
(2) Molecular weight: D-2520GPC Integrater, manufactured by Hitachi, Ltd.
(3) Differential scanning calorimeter (DSC): DSC50, manufactured by Shimadzu Corporation
(4) Thermogravimetric analyzer(TGA): TGA, manufactured by Shimadzu Corporation
(5) Melt spinning machine: 15φ small-type melt spinning machine, manufactured by Ohba Kikai Co., Ltd.
(6) Extending machine: small-type heat extending machine (with bath), manufactured by Ohba Kikai Co., Ltd.
(7) Measurement of strength: SHIMADZU AGS500B, manufactured by Shimadzu Corporation
(8) Dynamic viscoelasticity measuring device DMS-210, manufactured by Seiko Denshi Kogyo Co., Ltd.
(9) Biodegradability test: activated sludge, purchased on January 20, 1994, from Chemicals Inspection and Testing Institute

Using these apparatuses, measuring tests were carried out in accordance with "Degradability Test for Chemical Materials by Microorganisms" described in "Testing Method of New Chemical Substances" (Kanhogyo No. 5, Yakuhatsu No. 615, 49, Kikyoku No. 392, July 13, 1974) and Y. Doi, A. Segawa and M. Kunioka, Int. J. Biol. Macromol., 1990, Vol. 12, April, 106.

In the following examples, weight-average molecular weight, number-average molecular weight, glass transition point, melting point, and degradation point of the block copolymerized polyester compositions and polyesters used are referred to as Mw, Mn, Tg, Tm, and Td, respectively. Data on size, tensile strength, and extensibility are shown in Table 1.

The block copolymerized polyesters used in the examples were prepared in the following manner and isotacticity was calculated by a conventional method from the integration values of iso. peak and syn. peak of NMR spectrum.

### Synthesis Example 1

### Synthesis of block copolymerized polyester containing an isotactic-poly(3-hydroxybutyric acid) (hereinafter abbreviated as iso-P[(R)-3HB]) moiety and a poly(ε-caprolactone) (hereinafter abbreviated as PCL) moiety:

A 20 ml reaction vessel was charged with 1.72g (20 mmol) of (R)-BL(96%ee), 2.0 ml of toluene, and 5.6 mg (0.005 mmol) of 1-ethoxy-3-chlorotetrabutyl distannoxane and stirred in the presence of argon at 100°C for 5.5 hours. To the reaction solution was added 2.0 ml of toluene and immediately after was added 2.28 g(20 mmol) of ε-caprolactone for reaction for a predetermined period of time. The product was dissolved in chloroform and added to a mixed solution of methanol:hexane=1:2 and precipitated thereby to obtain a block copolymerized polyester containing an iso-P[(R)-3HB] moiety having 96% or more of isotacticity (iso-P[(R)-3HB]-b-PCL (1:1), Mn=162,000, Mw=251,000, Tg=-57°C, Tm=41.148°C, Td=293.381°C).

### Synthesis Example 2

### Synthesis of iso-P[(R)-3HB]-b-poly (L-lactic acid) (hereinafter abbreviated as iso-P[(R)-3HB])-b-PLLA:

A 500 ml reaction vessel was charged with 88.1g (1.02 mmol) of (R)-BL(96%ee) and 288mg (0.256 mmol) of 1-ethoxy-3-chlorotetrabutyl distannoxane, and stirred in the presence of argon at 100°C for one hour. To the reaction solution was added 140 ml of toluene and the mixture stirred further for 30 minutes. 147.5g (1.02 mmol) of L-lactic acid and 20 ml of toluene were added for reaction for a predetermined period of time. The product was dissolved in chloroform and added to a mixed solution of methanol:hexane=1:2 and precipitated, thereby to obtain a block copolymerized polyester containing an iso-P[(R)-3HB] moiety having 96 % or more of isotacticity (iso-P[(R)-3HB]-b-PLLA (1:1.8), Mn=172,000, Mw=300,000, Tg=41°C, Tm=154°C, Td=256.331°C).

### Example 1

### Spinning of a blend (1:1) of iso-P[(R)-3HB] and {iso-P[(R)-3HB]-b-PLLA}:

A polymer composition comprising a blend of iso-P[(R)-3HB] (Mn=348,000, Mw=760,000) and {iso-P[(R)-3HB]-b-PLLA} (1:1.8) prepared in Synthesis Example 2 and 0.1% by weight of talc was supplied to a melt spinning machine, and melt spun at a temperature of 176°C, followed by winding of the discharged filament at room temperature. The filament was then extended at 80°C by six times and wound to obtain a fiber having a diameter of 0.17 mm to 0.20 mm. The properties of the fiber obtained are shown in Table 1.

### Example 2

### Spinning of a blend (3:1:1) of iso-P[(R)-3HB], {iso-P[(R)-3HB]-b-PLLA}, and PLLA:

A polymer composition comprising a blend of iso-P[(R)-3HB] (Mn=354,000, Mw=658,000), {iso-P[(R)-3HB]-b-PLLA} (1:1.8) prepared in Synthesis Example 2, and PLLA (Mn=328,000, Mw=644,000), and 0.1% by weight of talc added thereto was supplied to a melt spinning machine and melt spun at a temperature of 186°C, followed by winding of the discharged filament at room temperature. The filament was then extended at 105°C by six times and wound to obtain a fiber having a diameter of 0.19 mm to 0.22 mm. The properties of the fiber obtained are shown in Table 1.

### Example 3

### Spinning of a blend (6:1:3) of iso-P[(R)-3HB], {iso-P[(R)-3HB]-b-PLLA}, and PLLA:

A polymer composition comprising a blend of iso-P[(R)-3HB] (Mn=261,000, Mw=593,000), iso-P[(R)-3HB]-b-PLLA (1:1.8) prepared in Synthesis Example 2, and PLLA (Mn=328,000, Mw=644,000), and 0.1% by weight of talc added thereto was supplied to a melt spinning machine, and melt spun at a temperature of 186°C, followed by winding of the discharged filament at room temperature. The filament was then extended at 105°C by six times and wound to obtain a fiber having a diameter of 0.19 mm to 0.22 mm. The properties of the fiber obtained are shown in Table 1.

### Example 4

### Spinning of a blend (1:1) of iso-P[(R)-3HB] and {iso-P[(R)-3HB]-b-PCL}:

A polymer composition comprising a blend of iso-P[(R)-3HB] (Mn=261,000, Mw=593,000) and iso-P[(R)-3HB]-b-PCL (1:1) prepared in Synthesis Example 1 was supplied to a melt spinning machine, and melt spun at a temperature of 186°C, followed by winding of the discharged filament. The filament was then extended at 25°C by seven times and wound to obtain a fiber having a diameter of 0.10 mm-0.12 mm. The properties of the fiber obtained are shown in Table 1.

**Table 1**

| Example | Extension Ratio S | Size (d) | Tensile Strength (kgf/mm²) | Extensibility (%) | Melting Point (°C) |
|---|---|---|---|---|---|
| 1 | 6 | 338 | 25.4 | 68.1 | 117 |
| 2 | 6 | 483 | 22.8 | 47.8 | 131 |
| 3 | 6 | 759 | 18.8 | 40.5 | 143 |
| 4 | 7 | 125 | 249 | 79.0 | 120 |

### Test Example 1

### Biodegradability test of fiber prepared in Example 1:

Activated sludge process was performed under the conditions of 500 ppm (600 ml), pH 6.0 to 7.0, and 25°C. 19 to 30 mg of the fiber obtained in Example 1 were added to a 50 ml flask and then the test was carried out using a shaking constant temperature bath, manufactured by Taitech Co., Ltd.

The loss in weight of the fiber after one week, two weeks, three weeks, and four weeks was respectively obtained. The amount and percentage of loss in weight are shown in Table 2.

### Test Examples 2 and 3

### Biodegradability test of fibers prepared in Examples 2 and 3:

Biodegradability tests were carried out on the fibers obtained in Examples 2 and 3 in the same manner as in Test Example 1. The obtained results are shown in Table 2.

**Table 2**

| Test Example | Loss in Weight (mg) | Loss in Weight (%) |
|---|---|---|
| 1 | 0.85 | 2.8 |
| 2 | 1.03 | 5.5 |
| 3 | 0.68 | 4.2 |

It is apparent from the results shown in Table 2 that the fiber obtained in Example 1 exhibits 0.85 mg of loss in weight after four weeks and the fibers obtained in Examples 1 and 3 exhibit 1.03 mg and 0.68 mg of loss in weight after four weeks, respectively.

According to the present invention, as described above, the disadvantage that a fiber comprising poly(3-hydroxybutyric acid) is low in extensibility and is brittle is eliminated, and a polyester fiber having a high strength, high biodegradability, and high hydrolyzability can be obtained in which the melting point and extensibility thereof can be controlled, by means of melt spinning of the polymer composition and extension thereof under suitable conditions.

Accordingly, the fiber according to the present invention is a biodegradable polyester fiber which has sufficient heat resistance to be used as a general material, has a melting point optionally changeable for medical use, and has high strength. Therefore, the fiber may be suitable for fishing materials such as fishing lines, fishing nets, and the like; agricultural materials such as moth-screening nets, bird-screening nets, planting nets, and the like; fabric or non-woven fabric for living materials such as women's sanitary goods, masks, wet tissue paper, underwear, towels, handkerchiefs, kitchen towels, diapers, and the like; and other industrial materials. When the fiber of the present invention is discarded, after it has been used, in an environment in which microorganisms are present, degradation and a decrease in strength thereof proceeds and the fiber is completely degraded within a certain period of time. By using the fiber of the present invention, no waste disposal apparatus is particularly required, and pollution and destruction of the environment may be prevented.

Since the fiber of the present invention is biocompatible, excellent in high dimensional stability in human tissue, and hydrolyzed and adsorbed in the body, it may be utilized for medical materials such as surgical sutures which need no extraction, surgical nets, suture reinforcement materials, and the like.

## Claims

1. A fiber comprising a biodegradable polymer, said fiber comprising a block copolymerized polyester represented by the following general formulae: 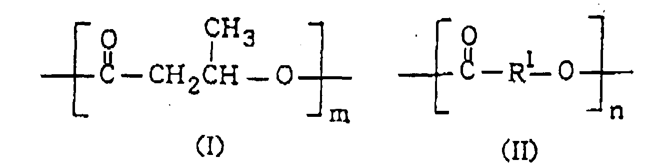 wherein, R¹ is a divalent hydrocarbon group having from 2 to 14 carbon atoms which may contain an oxygen atom or double bond; m and n are each an integer from 100 to 9,000, and m+n is from 200 to 10,000;
and in which the structural unit (I) consists of a stereospecific poly(3-hydroxybutyric acid); molar ratio of the structural unit (I) to (II) ranges from 10:90 to 90:10; and said block copolymerized polyester has a number average molecular weight in the range of 20,000 to 1,000,000.

2. A fiber comprising a biodegradable polymer composition, said fiber comprising
a block copolymerized polyester represented by the following general formulae: 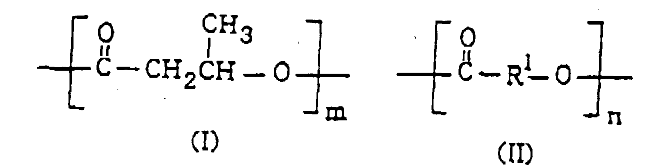 wherein, R¹ is a divalent hydrocarbon group having from 2 to 14 carbon atoms which may contain an oxygen atom or double bond; m and n are each an integer from 100 to 9,000, and m+n is from 200 to 10,000;
and in which the structural unit (I) consists of a stereospecific poly(3-hydroxybutyric acid);molar ratio of the structural unit (I) to (II) ranges from 10:90 to 90:10; and said block copolymerized polyester has a number average molecular weight in the range of 20,000 to 10,000; and
at least one polyester selected from the group consisting of poly(3-hydroybutyric acid) and a random copolymerized polyester containing 3-hydroxybutyric acid units.

3. A fiber comprising a biodegradable polymer composition according to claim 1 or 2, further comprising a polyester having a structure as defined for the structural unit (II) represented by said general formula.

4. A process for preparing a fiber comprising a biodegradable polymer composition according to anyone of claims 1 to 3, wherein melt spinning is carried out at a temperature from 150 to 220°C and extension of the fiber carried out at a temperature of from 10 to 150 °C.
